# EUROPÄISCHE PATENTSCHRIFT

(11) **EP 2 499 978 B1**
(45) Veröffentlichungstag und Bekanntmachung des Hinweises auf die Patenterteilung: **24.08.2016**
(21) Anmeldenummer: 12001845.2
(22) Anmeldetag: 19.03.2012
(51) Int. Cl.: A61B 17/14, B23D 51/10, B23D 61/00, B27B 19/00

(54) **Halterung für ein Oszillationssägeblatt**
Holder for an oscillation saw blade
Fixation pour une lame de scie à oscillations

(30) Priorität: 18.03.2011 DE 102011014497; 22.09.2011 DE 102011113866
(43) Veröffentlichungstag der Anmeldung: 19.09.2012
(73) Patentinhaber: WSEngineering GmbH & Co. KG, 78054 Villingen-Schwenningen (DE)
(72) Erfinder: Lay, Norbert, Dr., 71149 Bondorf (DE)
(74) Vertreter: Blumbach Zinngrebe

(56) Entgegenhaltungen:
- EP-A1- 0 722 802
- EP-A2- 1 422 009
- WO-A2-2011/083169
- DE-C1- 4 036 904
- DE-U1-202008 007 295
- US-A- 4 106 181
- US-A- 5 309 805

## Beschreibung

### Gebiet der Erfindung

Die Erfindung betrifft eine Halterung mit einem Oszillationssägeblatt sowie ein Oszillationssägewerkzeug.

### Hintergrund der Erfindung

Oszillationssägen sind bekannt. Es handelt sich dabei um ursprünglich für die medizinische Verwendung entwickelte Sägen, bei denen ein Sägeblatt, welches stirnseitig Zähne aufweist, über eine Antriebswelle in eine oszillierende Bewegung mit geringem Hub, insbesondere mit einem Winkel von weniger als 5°, versetzt wird. Im medizinischen Bereich ist es mit derartigen Werkzeugen möglich, Knochenmaterial oder Gips zu sägen, ohne dass Weichgewebe des Patienten beschädigt wird, da dieses auf Grund des geringen Hubs der Säge mitschwingen kann.

Das Dokument US 4,106,181 zeigt eine chirurgische Säge mit einem Schnellspannmechanismus, der einen Exzenter umfasst.

Aber auch im Handwerksbereich, insbesondere zur Bearbeitung von Holz, werden Oszillationssägewerkzeuge verwendet. Mit derartigen Oszillationswerkzeugen lassen sich beispielsweise auch in Ecken und Kanten Einstiche einbringen, was ansonsten mit kaum einem anderen Werkzeug möglich ist.

Darüber hinaus sind Oszillationssägen vielseitig verwendbar und können mit einer Vielzahl von verschiedenen Sägeblättern versehen werden. So ist es unter anderem auch möglich, diamantbesetzte Blätter zum Schneiden von Fliesen oder Schaber zum abrasiven Abtragen von Material aufzusetzen.

Oszillationssägen können sogar zum Schneiden von Metall verwendet werden. Beispielsweise beim Ausbau von Fenstern können die meist schwer zugänglichen Bolzen, mit denen das Fenster verankert ist, durchtrennt werden.

Die Anforderungen insbesondere an ein im Handwerksbereich verwendetes Oszillationssägeblatt sind daher hoch. Anders als im medizinischen Bereich wird von den Sägeblättern eine hohe Standzeit erwartet. Weiter sind zumindest teilweise die bearbeiteten Materialien deutlich härter, was sowohl die Belastung des Sägeblatts selbst als auch die Belastung des Antriebs, insbesondere an der Antriebswelle des Werkzeugs erhöht.

Unter anderem aus der Praxis bekannt sind daher Oszillationssägeblätter, welche aus zwei zusammen geschweißten Abschnitten unterschiedlichen Materials bestehen. Dabei wird für einen Basisabschnitt, welcher beispielsweise über eine formschlüssige Verbindung an einer Antriebswelle befestigt wird, ein relativ hartes Material verwendet, wohin gegen das Sägeblatt aus einem hieraus angeschweißten dünnen Streifen aus flexiblem Material besteht. Um auch in Ecken sägen zu können, sind derartige Sägeblätter in der Regel abgekröpft.

Unter anderem um auf diese aufwendig herstellbare Materialkombination verzichten zu können, wird in dem Dokument DE 10 2007 036 322 A1 vorgeschlagen, ein abgewinkeltes Sägeblatt in einer Aufnahme einzuspannen.

Nachteilig an sämtlichen bekannten Lösungen ist, dass zum Austausch des Sägeblatts entweder ein Werkzeug, beispielsweise zum Lösen einer Spannvorrichtung, verwendet werden muss oder Teile der Werkzeugmaschine entfernt werden müssen, um das Oszillationssägeblatt frei zu geben. Dies ist nicht nur umständlich, sondern kann beispielsweise bei der Verwendung auf Baustellen dazu führen, dass das Werkzeug bzw. Teile des Oszillationssägewerkzeugs herunter fallen und verloren gehen.

Eine werkzeuglose Halterung ließ sich aufgrund der hohen Stabilität, die eine solche Halterung bei der Verwendung im handwerklichen Bereich haben muss, aber bislang nicht hinreichend realisieren.

### Aufgabe der Erfindung

Der Erfindung liegt demgegenüber die Aufgabe zu Grunde, eine werkzeuglose Befestigung eines Oszillationssägewerkzeugs bereit zu stellen, welche auch für die Verwendung im handwerklichen Bereich geeignet ist und bei welcher der Benutzer lediglich mit Sägeblatt und Werkzeugmaschine hantieren muss, ohne beispielsweise Stifte oder ähnliches zum Herausnehmen des Sägeblatts zu entfernen.

### Zusammenfassung der Erfindung

Die Erfindung wird bereits durch eine Halterung für ein Oszillationssägeblatt nach Anspruch 1 gelöst.

Bevorzugte Ausführungsformen und Weiterbildungen der Erfindung sind den jeweiligen Unteransprüchen zu entnehmen.

Die Erfindung betrifft eine Halterung für ein Oszillationssägeblatt. Wie eingangs ausgeführt, handelt es sich dabei um ein Sägeblatt, welches in eine oszillierende Schwingung versetzbar ist und mit dem gerade Einschnitte senkrecht zur Antriebsachse eingefügt werden können. Hierzu umfasst das Oszillationssägeblatt stirnseitig typischerweise Zähne. Unter einem Sägeblatt im Sinne der Erfindung werden aber jegliche Blätter zum abrasiven Bearbeiten von Material, also beispielsweise auch Sägeblätter mit einer Diamantkörnung, mit der beispielsweise Fliesen bearbeitet werden können, oder Schaber mit unverzahnter Schneide verstanden.

Die Halterung umfasst Mittel zur Befestigung des Oszillationssägeblatts, welches ein Basisabschnitt umfasst, der dessen Befestigung dient.

Gemäß der Erfindung ist der Basisabschnitt des Oszillationssägeblatts in die Halterung einschiebbar und über eine werkzeuglos betätigbare Arretierung arretierbar.

Gemäß der Erfindung ist der Basisabschnitt des Sägeblatts mittels eines werkzeuglos bewegbaren Klemmelementes einspannbar. Das das Klemmelement wird über einen Exzenter bewegt. Der Erfinder hat herausgefunden, dass es mittels eines Exzenters möglich ist, eine form- und kraftschlüssige Verbindung herzustellen, welche das Sägeblatt derart festhält, dass die Halterung hohen Beanspruchungen stand hält und gleichzeitig ein Bewegen des Sägeblatts in der Halterung, welche die Schnittleistung des Werkzeugs reduziert, weitgehend verhindert wird.

Bei einer Weiterbildung der Erfindung wird der Basisabschnitt mittels eines Federelements, insbesondere mittels eines Elastomerelementes kraftschlüssig gehalten. Dabei wird vorzugsweise das Oszillationssägeblatt mittels des Exzenters gegen das Federelement gespannt. Insbesondere werden Federelemente aus Polyurethan verwendet. Derartige Federelemente sind langlebig und halten hohen Kräften stand. Die Federelemente haben in erster Linie die Aufgabe, Toleranzen des Sägeblatts zu kompensieren. Daher werden die Federelemente beim Einspannen des Sägeblatts nur geringfügig zusammengedrückt, insbesondere weniger als 1 mm, vorzugsweise weniger als 0,3 mm.

Bei einer bevorzugten Ausführungsform der Erfindung liegt der Basisabschnitt des Oszillationssägeblatts in einem verriegelten Zustand auf zumindest einer Seite, vorzugsweise beidseitig, in einem Anschlag der Halterung an. Der Basisabschnitt des Sägeblatts wird also an seiner quer zur Schnittrichtung verlaufenden Seite formschlüssig gehalten, wodurch ebenfalls mögliche Bewegungen des Sägeblatts reduziert werden. Diesbezüglich kommt der Halterung die Ausgestaltung eines Einspannmechanismus mittels Exzenters entgegen, der derart weit geöffnet werden kann, dass das Sägeblatt von vorne in die Halterung eingeschoben werden kann.

Dies ist, wie es bei einer Weiterbildung der Erfindung vorgesehen ist, auch bei Sägeblättern möglich, welche den im Folgenden noch näher beschriebenen Halteabschnitt aufweisen, der eine zusätzliche formschlüssige Verbindung bereit stellt.

Der Exzenter ist vorzugsweise mittels eines Hebels bewegbar, welcher in einem verriegelten Zustand in seiner Position gehalten wird. Hierzu kann insbesondere eine Rastfeder beziehungsweise eine Rastzunge verwendet werden, in welcher der Hebel im verriegelten Zustand einrastet. Da die Verriegelung des Hebels keine besonders hohen Kräfte aufnehmen muss, sind auch andere Ausführungsvarianten wie beispielsweise ein Magnet oder ein Druckknopf, welcher den Hebel im verriegelten zustand festhält, denkbar.

Der Basisabschnitt ist gegenüber einem Arbeitsabschnitt des Oszillationssägeblatts abgewinkelt. Aufgrund der abgewinkelten Ausgestaltung und einer formschlüssigen Verbindung sind die auf den Basisabschnitt quer zur Schnittrichtung wirkenden Kräfte gering, so dass eine werkzeuglose Arretierung bereit gestellt werden kann, welche keine sehr hohen Kräfte mehr aufnehmen muss.

Gemäß einer bevorzugten Ausführungsform der Erfindung ist die Aussparung, in welche das Oszillationssägeblatt geschoben wird, mindestens 1 cm vom Mittelpunkt der Antriebswelle einer Werkzeugmaschine beabstandet.

Der Halter umfasst vorzugsweise Mittel zum Befestigen an der Antriebswelle eines Oszillationssägeblatts, welche quer zur Schnittrichtung verlaufen.

Je nach verwendetem Sägewerkzeug kann dies beispielsweise ein einfacher Sechskant oder ein anderes Mittel zum formschlüssigen Befestigen auf der Antriebswelle sein. Über unterschiedlich geformte Befestigungsmittel kann so die Halterung gleichzeitig als Adapter zum Befestigen an der Werkzeugmaschine dienen.

Um eine verbesserte formschlüssige Befestigung des Sägeblatts in der Aussparung zu erreichen, weist diese bei einer Weiterbildung der Erfindung einen Basisabschnitt auf, an den sich ein abgewinkelter Arbeitsabschnitt sowie dem abgewinkelten Arbeitsabschnitt gegenüber ein abgewinkelter Halteabschnitt anschließen. Insbesondere ist das Sägeblatt U- oder treppenförmig ausgebildet, wobei im eingesetzten Zustand der Halteabschnitt an der Halterung anliegt, insbesondere indem dieser in einem Schlitz der Halterung angeordnet ist, welcher sich an den Schlitz in welchem der Basisabschnitt angeordnet ist, anschließt.

Insbesondere wenn, wie es ebenfalls bei einer bevorzugten Ausführungsform der Erfindung vorgesehen ist, Arbeitsabschnitt und Basisabschnitt einen stumpfen Winkel, insbesondere einen Winkel zwischen 100° und 150° einschließen, können so über die Anlage des Halteabschnitts an der Halterung hohe Drehmomente übertragen werden.

Es genügt dafür ein recht kurz ausgebildeter Halteabschnitt, insbesondere mit einer Länge von weniger als 1 cm, vorzugsweise weniger als 5 mm.

Der Basisabschnitt hat dagegen eine Länge zwischen 5 und 25 mm, vorzugsweise zwischen 10 und 20 mm, und der Arbeitsabschnitt eine Länge von 35 - 150 mm, bevorzugt von 45 - 100 mm.

Bei einer Ausführungsform der Erfindung wird die Aussparung der Halterung, in welche das Sägeblatt einschiebbar ist, zwischen einem Grundträger und einer an dem Grundträger befestigten, insbesondere angeschraubten Platte, ausgebildet. So wird zum einen die Herstellung der Halterung erleichtert.

Weiter kann die Platte vom Grundträger entfernt werden, um das Sägeblatt auch dann heraus nehmen zu können, wenn dieses sich beispielsweise aufgrund einer Beschädigung verklemmt hat oder dieses beispielsweise aufgrund von Verschmutzungen fest sitzt.

Der abgewinkelte Basisabschnitt sitzt vorzugsweise über seine gesamte Länge in der Aussparung der Halterung, um eine besonders haltbare formschlüssige Verbindung zu erreichen. Vorzugsweise sitzt dieser auch über seine gesamte Breite in der Aussparung, wobei sich versteht, dass randseitiger Überstand grundsätzlich die Funktion der erfindungsgemäßen Halterung nicht beeinträchtigt.

Die Erfindung betrifft insbesondere ein Oszillationssägewerkzeug zum Bearbeiten von Holz und/oder Metall, welches vorstehend beschriebene Halterung umfasst, die über einer oszillierend angetriebenen Antriebswelle in eine oszillierende Schwingung versetzt wird.

### Beschreibung der Zeichnungen

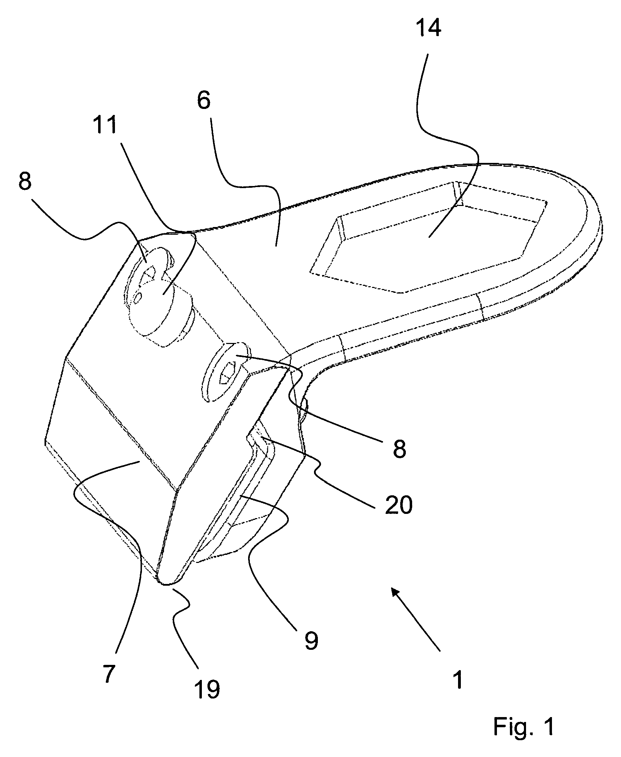

Die Erfindung soll im Folgenden Bezug nehmend auf schematisch dargestellte Ausführungsbeispiele anhand der Zeichnungen Fig. 1 bis Fig. 13 näher erläutert werden.

Fig. 1 zeigt eine perspektivische Ansicht einer nicht erfindungsgemäß ersten Ausführungsform einer Halterung 1, wobei in dieser Ansicht das Sägeblatt nicht eingesetzt ist.

Die Halterung 1 umfasst einen Grundträger 6, der Mittel zur Befestigung einer Antriebswelle 14, in diesem Fall als einfacher Sechskant ausgebildet, umfasst. Es versteht sich, dass die Mittel zur Befestigung an der Antriebswelle 14 eines Oszillationssägewerkzeugs je nach Maschine, für welche die dargestellte Halterung verwendet werden soll, unterschiedlich sind. So kann diese beliebige Formschlusselemente umfassen, um diese an der Antriebswelle einer Werkzeugmaschine anzukoppeln.

Weiter umfasst die Halterung 1 eine mittels der Schrauben 8 am Grundträger 6 befestigte Platte 7.

Zwischen der Platte 7 und dem Grundträger 6 ist eine schlitzförmige Aussparung 9 ausgebildet, durch welche das Oszillationssägeblatt (nicht dargestellt) seitlich in die Halterung 1 eingeschoben werden kann. Die Aussparung 9 steht zum Grundträger 6 abgewinkelt, so dass bei eingesetztem Sägeblatt durch die Halterung 6 eine Abkröpfung ausgebildet ist, die sicher stellt, dass mit dem Sägeblatt auch in Ecken Einstiche gesetzt werden können.

Um einen Formschluss, mittels dessen auch hohe Kräfte übertragen werden können, auszubilden, umfasst die Aussparung 9 einen abgewinkelten Abschnitt 20 für einen Halteabschnitt des Sägeblatts. Das Sägeblatt (nicht dargestellt) ist in diesem Ausführungsbeispiel mithin abschnittsweise U-förmig gestaltet, so dass es aufgrund der Anlage in dem Abschnitt 20 nicht nach vorne heraus rutschen kann.

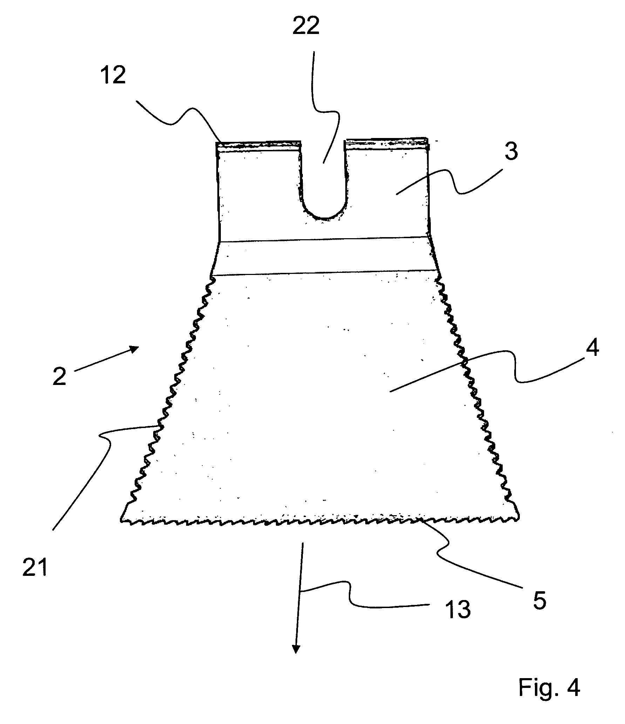

Der abgewinkelte Basisabschnitt des Sägeblatts (3 in Fig. 4) liegt in eingesetztem Zustand an der Anlagefläche 19 an. Somit ist das Sägeblatt durch die Aussparung 9 lediglich in die Einschubrichtung nicht formschlüssig fixiert.

Um das eingeschobene Sägeblatt zu fixieren, umfasst die Halterung ein Betätigungsmittel 11 in Form eines Knopfes, über welches zum Einsetzen ein Eingriffelement (nicht dargestellt) herab gedrückt wird, welches in nicht betätigtem Zustand formschlüssig in eine entsprechend geformte Aussparung des Sägeblattes eingreift.

Zum Einsetzen des Sägeblattes braucht der Benutzer somit nur das Betätigungsmittel 11 zu drücken und das Sägeblatt einzuschieben. Das Betätigungsmittel 11 wird durch Federkraft zurück gehalten und lässt das Eingriffelement in die Aussparung des Sägeblatts einrasten, sobald dieses mittig positioniert ist.

So kann zum einen auf eine Verwendung von Werkzeug verzichtet werden. Zum anderen hat der Benutzer nur zwei Bauteile in den Händen zu halten, nämlich die Oszillationssägemaschine (nicht dargestellt) mit der Halterung 1 sowie das Sägeblatt. Das Einsetzen des Sägeblattes kann daher auch ohne Bauteile abzulegen freihändig erfolgen.

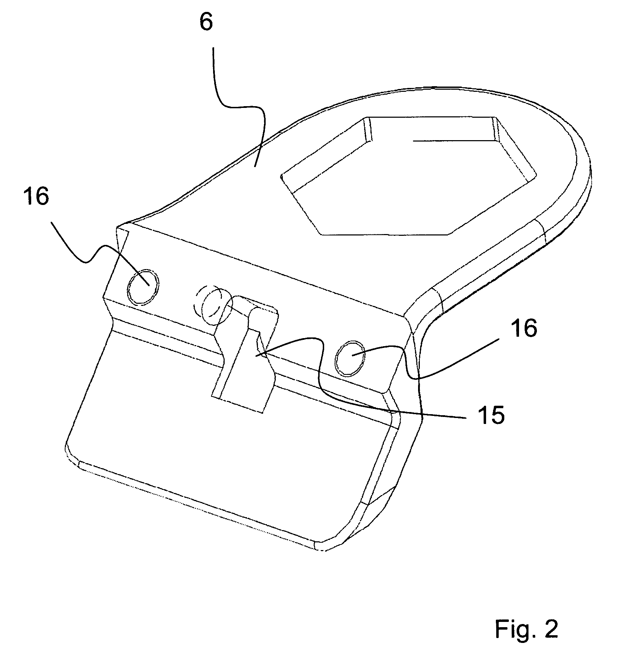

Fig. 2 zeigt eine perspektivische Ansicht des Grundträgers 6, wobei in dieser Ansicht die Platte mit dem Betätigungsmittel (7 in Fig. 1) abgenommen ist.

Zum Anschrauben der Platte umfasst der Grundträger 6 die Gewinde 16. Zusätzlich können Platte und Grundträger mit Stiften verbunden werden.

Weiter umfasst der Grundträger eine in diesem Ausführungsbeispiel im Wesentlichen rechteckig ausgebildete Ausnehmung 15, in welcher das Eingriffelement zum Fixieren des Sägeblattes ruht (nicht dargestellt).

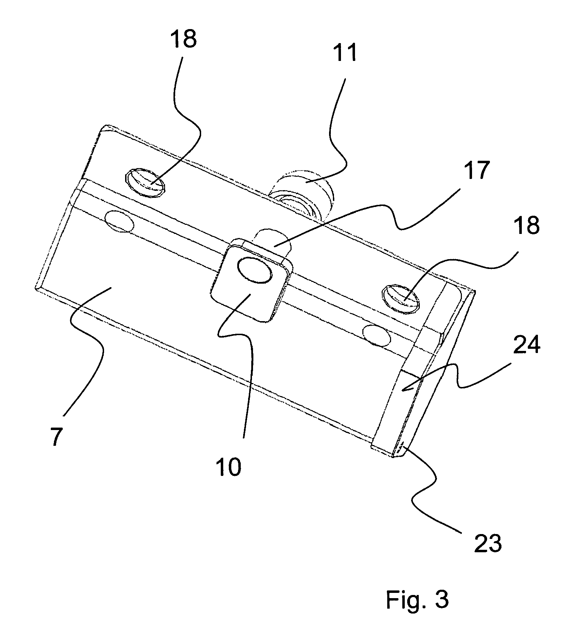

Fig. 3 zeigt eine perspektivische Ansicht der Platte 7, welche mittels Schrauben, die durch die Bohrungen 18 greifen, am Grundträger (6 in Fig. 2) befestigt wird.

Das Betätigungsmittel 11 ist über ein Stift 17 in der Platte 7 geführt.

An den Stift 17 schließt sich das Eingriffelement 10 zum Fixieren des eingeschobenen Sägeblattes an.

In der Grundposition wird das Eingriffelement 10 über eine Feder (nicht dargestellt) in der Position gehalten, in welcher das Eingriffelement 10 das Sägeblatt fixiert.

Die Feder kann beispielsweise zwischen dem Betätigungsmittel 11 und der Platte 7 auf dem Stift 17 angeordnet oder in der Ausnehmung des Grundträgers (15 in Fig. 2) eingesetzt sein.

Quer zu den Bohrungen 1 sind Platte 7 und Grundträger (15 in Fig. 2) über zumindest einen Stift 23 verbunden, um so höhere Kräfte aufnehmen zu können.

In diesem Ausführungsbeispiel ist auf einer Seite der Halterung ein Anschlag 24 vorgesehen, so dass das Sägeblatt nur von einer Seite in die Halterung geschoben werden kann, durch den Anschlag 24 aber in definierter Position zur Anlage kommt. Es versteht sich, dass der Anschlag nicht zwingend, wie hier dargestellt, Teil der Platte 7 sein muss, sondern dass dieser beispielsweise auch Teil des Grundträgers (15 in Fig. 2) sein kann.

Insbesondere bei Verwendung eines Anschlags kann in einer hier nicht dargestellten Ausführungsform das Eingriffelement auf der Einschubseite auch angeschrägt sein, so dass dieses beim Einschieben des Sägeblatts ohne eine Betätigung des Betätigungsmittels weggedrückt wird, so dass das Sägeblatt in seine Entstellung kommt und das Betätigungsmittel sodann selbständig einrastet. Bei Verwendung eines Anschlags genügt es, wenn das Eingriffelement die Aussparung auf der dem Sägeblatt abgewandten Seite fixiert. So ist beim Einsetzen des Sägeblatts keine Betätigung des Betätigungsmittels erforderlich, sondern das Sägeblatt rastet selbständig ein und nur zum Entriegeln und Herausnehmen des Sägeblatts ist ein Betätigen des Betätigungsmittels erforderlich.

Fig. 4 zeigt ein Ausführungsbeispiel eines Sägeblattes zum Einsetzen in die in Fig. 1 dargestellte Halterung.

Das Sägeblatt umfasst einen Arbeitsabschnitt 4 mit einer stirnseitigen Verzahnung 5, über die Einstiche gesägt werden können. Die Schnittrichtung ist mit Pfeil 13 gekennzeichnet. Das hier dargestellte Sägeblatt weist auch eine seitliche Verzahnung 21 auf, welche in erster Linie dem Ausräumen von Spänen dient und auf welche auch verzichtet werden kann.

An den Arbeitsabschnitt 4 schließt sich ein abgewinkelter Basisabschnitt 3 an. Arbeitsabschnitt 4 und Basisabschnitt 3 schließen vorzugsweise einen stumpfen Winkel, insbesondere einen Winkel zwischen 110° und 150° ein.

Gegenüber des Arbeitsabschnitts 4 schließt sich an den Basisabschnitt 3, welcher in die in Fig. 1 dargestellte Aussparung geschoben wird, ein kurzer Halteabschnitt 12 an, welcher ebenfalls abgewinkelt ist. Durch die doppelte Abwinklung, also den abgewinkelten Arbeitsabschnitt 4 und den abgewinkelten Halteabschnitt 12, ist das Sägeblatt beim Einschieben bis auf die Einschubrichtung formschlüssig fixiert.

Arbeitsabschnitt 4 und Halteabschnitt 12 sind vorzugsweise in dieselbe Richtung abgewinkelt, mithin ist das Sägeblatt im Wesentlichen U-förmig gebogen, wobei der Halteabschnitt 12 erheblich kürzer ausgebildet ist als der Arbeitsabschnitt 4.

Zum Fixieren des eingeschobenen Sägeblattes weist dieses eine Aussparung 22 auf.

Die Aussparung 22 ist in diesem Ausführungsbeispiel nach oben, also in Richtung des Halteabschnitts 12, geöffnet. Im eingesetzten Zustand ist das entsprechende Eingriffelement (10 in Fig. 3) derart ausgebildet, dass es zumindest auf den der Einschubrichtung zugewandten Seiten der Aussparung 22 anliegt und so das Sägeblatt 2 im eingeschobenen Zustand fixiert.

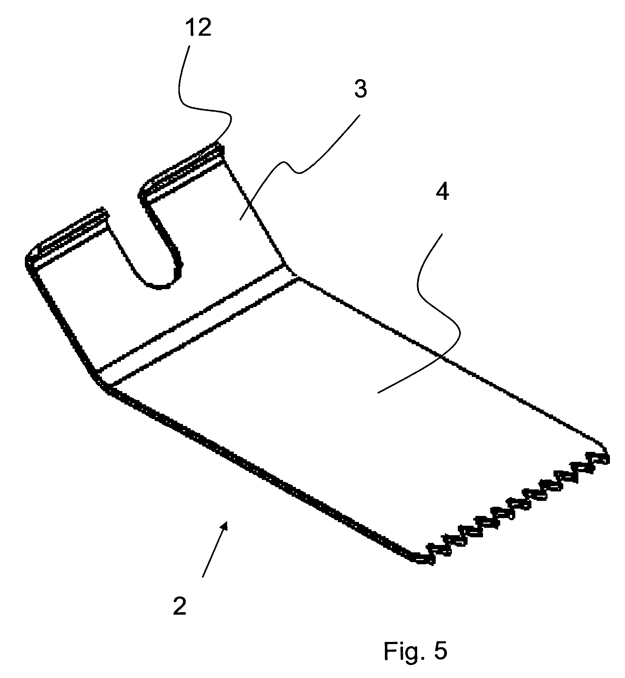

Fig. 5 zeigt eine schematische perspektivische Ansicht eines Oszillationssägeblatts 2.

Zu erkennen ist, dass das Oszillationssägeblatt 2 einen als Platte ausgebildeten Arbeitsabschnitt 4 aufweist, an welchen sich ein Basisabschnitt 3, der der Befestigung des Oszillationssägeblatts 2 dient, anschließt. Arbeitsabschnitt 4 und Basisabschnitt 3 schließen einen stumpfen Winkel ein.

An den ebenfalls plattenförmigen Basisabschnitt (3) schließt sich im oberen Bereich ein abgewinkelter Halteabschnitt 12 an, bei dem sich eine weitere formschlüssige Befestigung des Sägeblatts erzielen lässt.

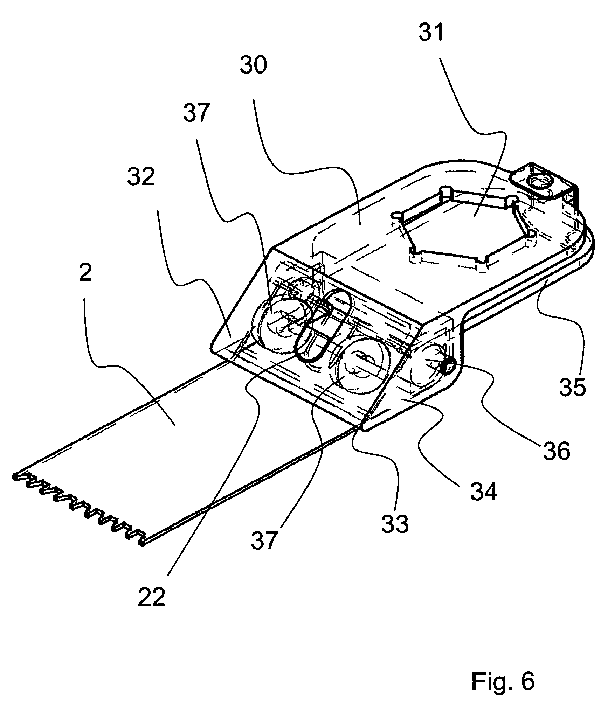

Bezugnehmend auf Fig. 6 bis Fig. 11 soll anhand eines schematisch dargestellten Ausführungsbeispiels eine gegenüber den Figuren Fig. 1 bis Fig. 3 alternative Ausführungsform einer Halterung für ein Oszillationssägeblatt näher erläutert werden.

Fig. 6 zeigt eine schematische perspektivische Ansicht einer erfindungsgemäßen Halterung 30 für ein Oszillationssägeblatt, bei welcher das in Fig. 5 dargestellte Oszillationssägeblatt 2 eingesetzt ist.

Die Halterung 30 umfasst ein Mittel zur Befestigung an der Antriebswelle eines Oszillationssägewerkzeugs 31, welche in diesem Ausführungsbeispiel als Sechskant ausgebildet ist. Der plattenförmig ausgebildete Arbeitsabschnitt des Oszillationssägeblatts 2 verläuft senkrecht zur Mittelachse des sechskantförmigen Mittels zur Befestigung an der Antriebswelle 31.

In diesem Ausführungsbeispiel wird das Oszillationssägeblatt 2 mittels eines Exzenters 36 in der Halterung eingespannt. Hierzu ist der Exzenter 36 mit einem Hebel 35 verbunden bzw. einstückig ausgebildet, welcher in einem verriegelten Zustand in die hier dargestellte Position bewegt wird. Durch Bewegung des Exzenters 36 in diese Position wird ein Klemmelement 34 nach vorne bewegt und spannt den Basisabschnitt des Oszillationssägeblatts 2 in der Halterung 30 ein. Das Klemmelement 34 hat ein im Wesentlichen rampenförmiges Profil, welches mit dem abgewinkelten Basisabschnitt des Oszillationssägeblatts 2 korrespondiert und so für eine vollflächige Anlage des Basisabschnitts sorgt.

Über das Klemmelement wird das Sägeblatt gegen zwei Federelemente 37, welche als im Wesentlichen runde Elastomerelemente aus Polyurethan ausgebildet sind, gepresst.

Diese Federelemente können auf dem dem Klemmelement 34 gegenüberliegendem Abschnitt der Halterung 30 aufgeklebt oder formschlüssig befestigt, beispielsweise eingerastet werden.

Die Federelemente 37 kompensieren möglicherweise vorhandene Fertigungstoleranzen des Oszillationssägeblatts 2.

Randseitig liegt der Basisabschnitt des Oszillationssägeblatts 2 oberhalb des Arbeitsabschnitts an den Anschlägen 32 und 33 an. Das Oszillationssägeblatt wird also beidseitig geführt, wodurch die Gefahr einer Bewegung des Oszillationssägeblatts 2 in der Halterung 30 beim Sägen reduziert wird.

Weiter besitzt das Oszillationssägeblatt 2 eine Aussparung 22 welche zumindest teilweise im verriegelten Zustand an einem korrespondierenden Eingriffelement der Halterung 30 anliegt. Über diese Aussparung 22 und der korrespondierenden Halterung wird somit eine weitere formschlüssige Verbindung im verriegelten Zustand bereit gestellt.

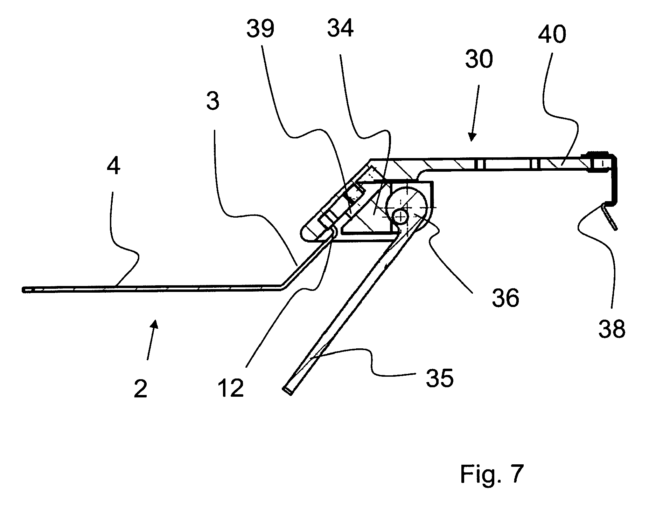

Fig. 7 zeigt eine schematische Schnittansicht der in Fig. 6 dargestellten Halterung, wobei in dieser Ansicht das Oszillationssägeblatt 2 nicht eingespannt ist.

In dieser Darstellung ist der Hebel 35 nach unten geklappt, so dass über den Exzenter 36 das rampenförmige Klemmelement 34 in dieser Ansicht nach rechts bewegt wurde.

Durch diese Bewegung wird ein Kanal 39 freigegeben, in welchen der Basisabschnitt 3 des Oszillationssägeblatts (2) eingeschoben werden kann. Der Kanal 39 ist dabei sogar so groß, dass der abgewinkelte Halteabschnitt 12 des Oszillationssägeblatts 2 durch den Kanal 39 geführt werden kann. Dieser Halteabschnitt 12 dient in einem verriegelten Zustand der zusätzlichen formschlüssigen Befestigung des Oszillationssägeblattes 2. Arbeitsabschnitt 4 des Oszillationssägeblatts 2 und Oberteil 40 der Halterung 30 verlaufen im eingesetzten Zustand parallel zueinander und senkrecht zur Antriebswelle des Oszillationssägewerkzeugs (nicht dargestellt). So können Einstiche auch in Ecken eingearbeitet werden.

Rückseitig, also gegenüber dem Oszillationssägeblatt, ist an der Halterung 30 eine Rastfeder 38 angebracht, in welcher der Hebel 35 im verriegelten Zustand einrastet. Diese Rastfeder 38 besteht vorzugsweise aus Federstahl und kann beispielsweise an das Oberteil 40 der Halterung genietet werden. Durch ein nach Hinten ziehen der Rastfeder 38 kann der Hebel 35 freigegeben und wieder in den hier dargestellten Zustand bewegt werden.

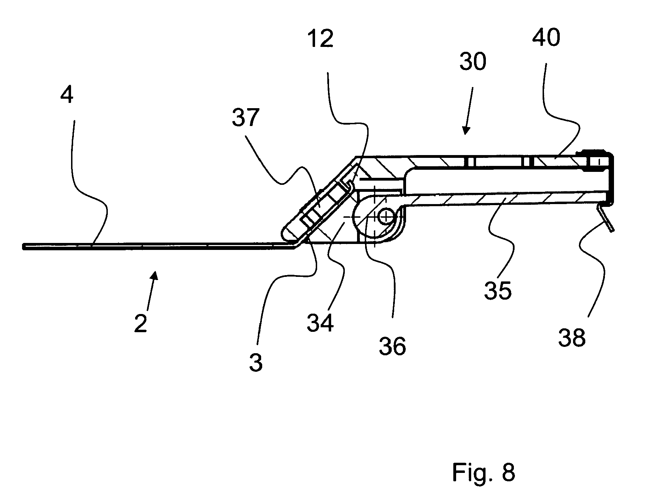

Fig. 8 zeigt eine weitere Schnittansicht, bei der nunmehr das Oszillationssägeblatt 2 in die Halterung 30 eingespannt ist.

Zu erkennen ist, dass aufgrund einer Bewegung des Hebels in 35 in Richtung des Oberteils 40 der Halterung 30 das Klemmelement 34 in Richtung des Basisabschnitts 3 des Oszillationssägeblatts 2 vorgeschoben wurde, dass nunmehr der Basisabschnitt 3 zwischen Klemmelement und Federelement 37 eingespannt ist.

Der abgewinkelte Halteabschnitt 12 des Oszillationssägeblatts 2 liegt an einer korrespondierenden Kante der Halterung 30 an und bildet eine zusätzliche formschlüssige Verbindung.

In dem verriegelten Zustand ist das Klemmelement 34 in seine vorderste Position geschoben und der Hebel 35, welcher sich im Wesentlichen parallel zum Oberteil 40 der Halterung 30 erstreckt, ist in der Rastfeder 38 eingespannt.

Im montierten Zustand kann sich nunmehr zwischen dem Hebel 35, der in diesem Ausführungsbeispiel in etwa der Kontur des Oberteils 40 folgt, und dem Oberteil beispielsweise der Kopf einer Befestigungsmutter befinden, mit welcher die Halterung 30 an der Antriebswelle des Oszillationssägewerkzeugs (nicht dargestellt) befestigt wird.

Alternativ ist aber auch denkbar, den Hebel 35 mit einer Aussparung zu versehen, durch welche eine Mutter oder ähnliches geführt werden kann. In diesem Fall könnte der Abstand zwischen Hebel 35 und Oberteil 40 weiter reduziert werden.

Zu erkennen ist in dieser Ansicht, dass der Arbeitsabschnitt des Oszillationssägeblatts 2 vom Oberteil 40 der Halterung aus gesehen am weitesten beabstandet ist. Es ragen also keine Bauteile unterhalb des Arbeitsabschnitts 4 des Oszillationssägeblatts 2 hervor, so dass dieses zum Setzen von Einstichen an Kanten verwendet werden kann.

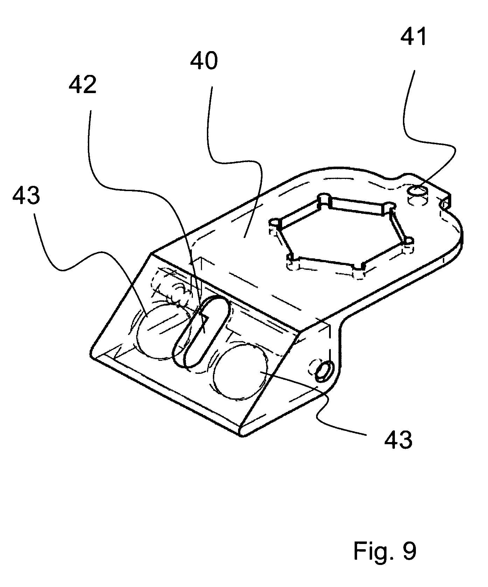

Fig. 9 zeigt eine perspektivische Ansicht eines Oberteils 40 der Halterung.

Das Oberteil weist rückseitig eine Bohrung 41 oder ein Gewinde zum Befestigen der Rastfeder (38 in Fig. 8) auf.

Am vorderen Teil, gegen den das Klemmelement fährt, weist das Oberteil 40 eine Ausnehmung 42 auf, in welche ein korrespondierendes Eingriffelement des Klemmelements bündig eingeschoben werden kann. Dabei liegt das korrespondierende Eingriffelement des Klemmelements ebenfalls bündig an den Seiten der mittigen Ausnehmung des Sägeblatts an. Die Ausnehmung 42 kann sowohl als Durchgangsloch als auch als Sackloch ausgebildet sein.

Das Oberteil 40 weist zwei weitere Ausnehmungen 43 auf, welche als Sacklöcher ausgebildet sind und welche zur Aufnahme der als Elastomerelemente ausgebildeten Federelemente (37 in Fig. 8) dienen.

In den Ausnehmungen 43 befestigt, beispielsweise eingeklebt, halten die Federelemente besser und es kann für das Ausdehnen und Zusammenziehen des Federelementes eine längere Strecke bereitgestellt werden.

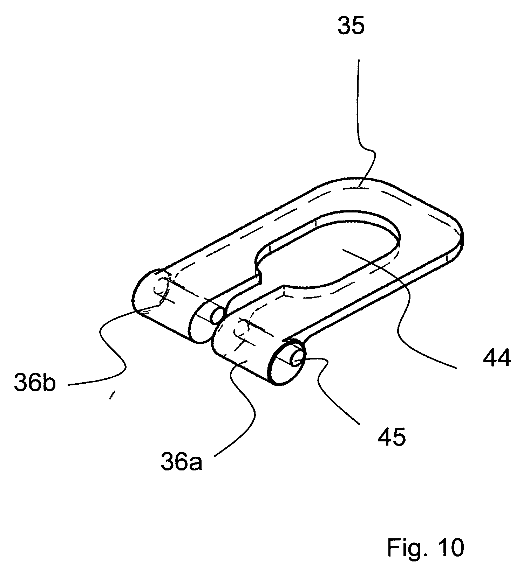

Fig. 10 zeigt eine perspektivische Ansicht des Hebels 35 mit dem Exzenter.

Der Hebel 35 weist eine mittige Ausnehmung 44 auf, durch die beispielsweise ein Befestigungsmittel zum Befestigen der Halterung an einer Antriebswelle hindurchragen kann.

Weiter umfasst der Hebel den Exzenter, welcher aus den beiden Exzenterabschnitten 36 a und 36 b besteht. Zur Ausbildung eines Exzenterlagers haben die Exzenterabschnitte 36 a und 36 b eine deaxial angebrachte Bohrung 45 durch die zum Lagern des Exzenters am Oberteil der Halterung ein Stift geführt werden kann.

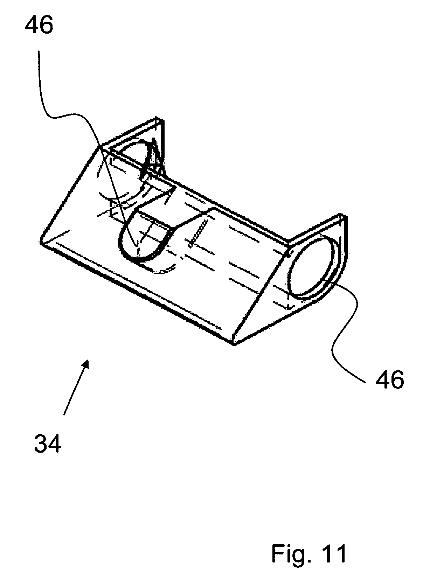

Die Aufteilung des Exzenters in zwei Abschnitte ermöglicht, dass der Hebel 35 wie eine Feder wirkt, so dass die Exzenterabschnitte 35a, 35b zur Montage des Exzenters am Oberteil zusammengedrückt werden können. Fig. 11 zeigt eine perspektivische Ansicht des Klemmelementes 34. Das Klemmelement 34 weist zwei Exzenterlager 46 auf, in welche das randseitig überstehende Stück der beiden Exzenterabschnitte (36 a und 36 b in Fig. 10) eingerastet werden kann.
Weiter weist das Klemmelement ein Eingriffelement 46 für die mittige Aussparung des Sägeblatts auf, welche beim Vorschieben des Klemmelements 34 auch in der Ausnehmung des Oberteils (42 in Fig. 9) randseitig zur Anlage kommt und so auch eine formschlüssige Befestigung des Klemmelements am Oberteil der Halterung bereitstellt.

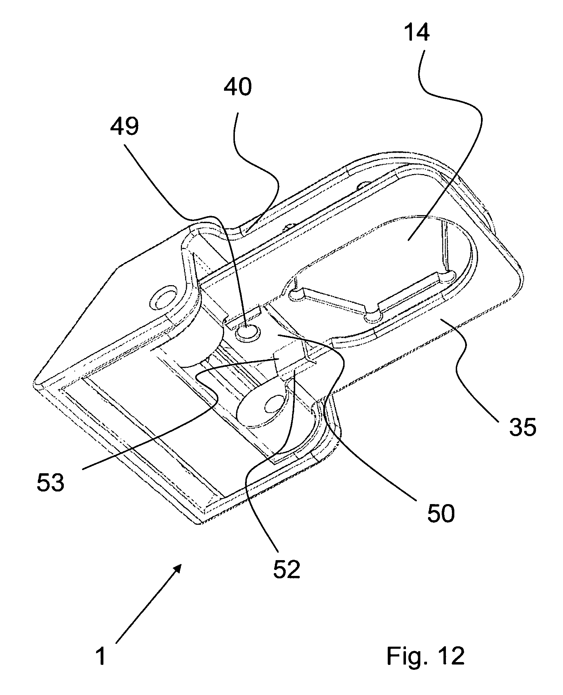

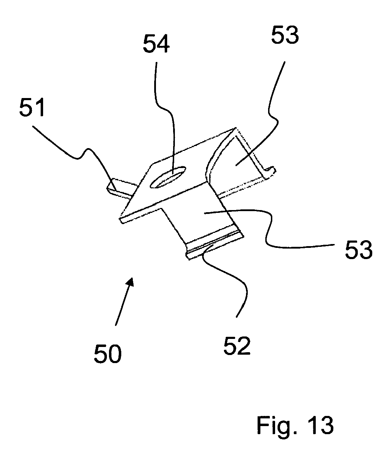

Bezug nehmend auf Fig. 12 und Fig. 13 soll eine weitere Ausführungsform der Erfindung erläutert werden, bei welcher der Mechanismus, um den Hebel 35 festzulegen, gegenüber der in den Figuren Fig. 6 bis Fig. 11 gezeigten Ausführungsform alternativ ausgebildet ist. Bis auf den Mechanismus zum Festlegen des Hebels entspricht die in den Figuren Fig. 12 und Fig. 13 gezeigte Ausführungsform somit der Ausführungsform gemäß Fig. 6 bis Fig. 11.

Fig. 11 zeigt eine perspektivische Ansicht einer Halterung 1 für ein Oszillationssägeblatt (nicht dargestellt), welche eine mit einem Exzenter versehenen schwenkbaren Hebel 35 aufweist.

Im Unterschied zu der in Fig. 6 bis Fig. 11 dargestellten Ausführungsform ist hier nicht am hinteren Ende der Halterung 1 sondern zwischen Sägeblattaufnahme und dem Mittel zur Befestigung an der Antriebswelle 14 eine Feder 50 angeordnet, die mittels eines Befestigungsmittels 49 am Oberteil angebracht ist. Bei dem Befestigungsmittel 49 kann es sich beispielsweise um eine Niet, Schraub- oder Schweißverbindung handeln.

Die Feder 50 weist zwei gegenüberliegende Flügel 53 auf. An den Flügeln 53 sind Rasthaken 52 angeordnet.

Sobald der Hebel 35 zum Festsetzen des Sägeblatts 35 in die Endstellung geschwenkt wird, können die Rasthaken 52 zunächst durch die als Federn wirkenden Flügel 53 ausweichen. Sodann rasten die Rasthaken 52 an der Unterseite des Hebels 35 ein.
Die Rasthaken 52 können beispielsweise als umgebogener Abschnitt der aus Federstahl bestehenden Flügel 53 ausgebildet sein.

Zum Herausnehmen des Sägeblatts (nicht dargestellt) kann der Hebel 35 gegen den Widerstand der Rasthaken 52 geschwenkt werden, wobei wiederum die Flügel 53 einfedern indem sich diese aufeinander zubewegen.

Diese Ausführungsform ermöglicht eine noch komfortablere Ausbildung des Festlegungsmechanismus, da ein hinteres Ende des Hebels 35 von der Feder 50 beabstandet ist. Aufgrund dieser Hebelwirkung ist eine geringere Kraft zum Lösen des als Feder ausgebildeten Festlegungsmechanismus erforderlich.

Fig. 13 zeigt eine perspektivische Ansicht der in Fig. 12 dargestellten Feder 50.

Zu erkennen sind die gegenüberliegenden Flügel 53 mit den Rasthaken 52.

Eine Grundplatte der Feder 50 weist eine Bohrung 54 auf, um die Feder 50 zu befestigen. Es versteht sich, dass der Begriff Bohrung im geometrischen Sinne verstanden wird und dass die Bohrung eine beliebige Ausgestaltung haben kann.

Weiter zu erkennen ist ein Fortsatz 51, welcher im montierten Zustand an einer korrespondierenden Fläche des Oberteils der Halterung (40 in Fig. 12 anliegt) und zusätzlich die mechanische Stabilität erhöht.

Durch die Erfindung konnte ein werkzeugloses Befestigungssystem für Oszillationssägeblätter ermöglicht werden, welches auch den Belastungen im Handwerksbereich gewachsen ist und bei welchem der Benutzer lediglich mit Werkzeug und Sägeblatt hantieren muss.

### Bezugszeichenliste

1 Halterung
2 Oszillationssägeblatt
3 Basisabschnitt
4 Arbeitsabschnitt
5 Verzahnung
6 Grundträger
7 Platte
8 Schrauben
9 Aussparung
10 Eingriffelement
11 Betätigungsmittel
12 Halteabschnitt
13 Pfeil
14 Mittel zur Befestigung an der Antriebswelle
15 Ausnehmung
16 Gewinde
17 Stift
18 Bohrung
19 Anlagefläche
20 Abschnitt für Halteabschnitt
21 seitliche Verzahnung
22 Aussparung
23 Stift
24 Anschlag
30. Halterung
31. Mittel zur Befestigung an der Antriebswelle
32. Anschlag
33. Anschlag
34. Klemmelement
35. Hebel
36. Exzenter
37. Federelement
38. Rastfeder
39. Kanal
40. Oberteil
41. Bohrung
42. Ausnehmung
43. Ausnehmung
44. Ausnehmung
45. Bohrung
46. Exzenterlager
47. Eingriffelement
49. Befestigungsmittel
50. Feder
51. Fortsatz
52. Rasthaken
53. Flügel
54. Bohrung

## Patentansprüche

1. Halterung (1) für ein Oszillationssägeblatt (2), umfassend Mittel zur Befestigung des Oszillationssägeblatts (2), mit einem Oszillationssägeblatt, welches einen Basisabschnitt (3) umfasst, welcher in eine Aussparung (9) der Halterung (1) einschiebbar ist und über eine werkzeuglos betätigbare Arretierung arretierbar ist, und mit einem Exzenter; durch ein rampenförmiges Klemmelement gekennzeichnet, und dadurch, dass der Basisabschnitt (3) des Oszillationssägeblatts (1) gegenüber einem Arbeitsabschnitt (4) abgewinkelt ist und mittels des über den Exzenter (36) bewegbaren rampenförmigen Klemmelementes (34) einspannbar ist.

2. Halterung für ein Oszillationssägeblatt nach einem dem vorstehenden Anspruch, **dadurch gekennzeichnet, dass** der Basisabschnitt (3) mittels zumindest eines Federelements (37), insbesondere mittels eines Elastomerelements, in einem verriegelten Zustand kraftschlüssig gehalten wird.

3. Halterung für ein Oszillationssägeblatt nach einem der vorstehenden Ansprüche, **dadurch gekennzeichnet, dass** der Basisabschnitt (3) auf zumindest einer Seite, vorzugsweise beidseitig, an einem Anschlag (32, 33) der Halterung anliegt.

4. Halterung für ein Oszillationssägeblatt nach einem der vorstehenden Ansprüche, **dadurch gekennzeichnet, dass** der Exzenter (36) mittels eines Hebels (35) bewegbar ist, welcher in einem verriegelten Zustand in seiner Position gehalten wird, insbesondere mittels einer Rastfeder (38).

5. Halterung für ein Oszillationssägeblatt nach einem der vorstehenden Ansprüche, **dadurch gekennzeichnet, dass** die Halterung Mittel zur Befestigung (14) an einer quer zur Schnittrichtung verlaufenden Antriebswelle aufweist.

6. Halterung für ein Oszillationssägeblatt nach dem vorstehenden Anspruch, **dadurch gekennzeichnet, dass** der Basisabschnitt (3) des Sägeblatts in der Halterung (1) in einem verriegelten Zustand formschlüssig anliegt.

7. Halterung für ein Oszillationssägeblatt nach einem der vorstehenden Ansprüche, **dadurch gekennzeichnet, dass** ein abgewinkelter Basisabschnitt (3) des Sägeblatts über seine gesamte Länge in der Aussparung der Halterung (1) eingeschoben ist.

8. Oszillationssägewerkzeug zum Bearbeiten von Holz und/oder Metall, umfassend eine oszillierend angetriebene Antriebswelle und eine Halterung (1) nach einem der vorstehenden Ansprüche.

## Claims

1. Holder (1) for an oscillating saw blade (2), comprising means for fastening the oscillating saw blade (2), having an oscillating saw blade which comprises a base portion (3) which can be pushed into a recess (9) in the holder (1) and can be locked by means of a lock which can be actuated without the use of tools,
and having a cam; **characterised by** a ramp-like clamping element and in that the base portion (3) of the oscillating saw blade (1) is angled with respect to a working portion (4) and can be clamped by means of the ramp-like clamping element (34) which can be moved via the cam (36).

2. Holder for an oscillating saw blade as claimed in the preceding claim, **characterised in that** the base portion (3) is held in a non-positive manner in a locked state by means of at least one spring element (37), in particular by means of an elastomeric element.

3. Holder for an oscillating saw blade as claimed in any one of the preceding claims, **characterised in that** the base portion (3) lies against a stop (32, 33) of the holder on at least one side, preferably both sides.

4. Holder for an oscillating saw blade as claimed in any one of the preceding claims, **characterised in that** the cam (36) can be moved by means of a lever (35) which, in a locked state, is held in its position, in particular by means of a catch spring (38).

5. Holder for an oscillating saw blade as claimed in any one of the preceding claims, **characterised in that** the holder has means for fastening (14) to a drive shaft extending transversely to the direction of cut.

6. Holder for an oscillating saw blade as claimed in the preceding claim, **characterised in that** the base portion (3) of the saw blade lies in a positive-locking manner in the holder (1) in a locked state.

7. Holder for an oscillating saw blade as claimed in any one of the preceding claims, **characterised in that** an angled base portion (3) of the saw blade is pushed over its entire length in the recess in the holder (1).

8. Oscillating saw tool for processing wood and/or metal, comprising a drive shaft driven in an oscillating manner and a holder (1) as claimed in any one of the preceding claims.

## Revendications

1. Elément de retenue (1) pour une lame de scie à oscillations (2), comportant des moyens permettant la fixation de la lame de scie à oscillations (2), comprenant une lame de scie à oscillations, laquelle présente une partie de base (3), laquelle peut être introduite dans un évidement (9) de l'élément de retenue (1) et peut être bloquée par l'intermédiaire d'un dispositif d'arrêt pouvant être actionné sans outil,
et comprenant un excentrique, ledit élément de retenue étant **caractérisé par** un élément de serrage en forme de rampe, et en ce que la partie de base (3) de la lame de scie à oscillations (1) est coudée par rapport à une partie de travail (4) et peut être maintenue par serrage au moyen de l'élément de serrage (34) en forme de rampe pouvant être déplacé par l'intermédiaire de l'excentrique (36).

2. Elément de retenue pour une lame de scie à oscillations selon l'une quelconque des revendications précédentes, **caractérisé en ce que** la partie de base (3) est retenue à force au moyen d'au moins un élément ressort (37), en particulier au moyen d'un élément élastomère, dans un état verrouillé.

3. Elément de retenue pour une lame de scie à oscillations selon l'une quelconque des revendications précédentes, **caractérisé en ce que** la partie de base (3) repose sur au moins une face, de préférence sur les deux faces, contre une butée (32, 33) de l'élément de retenue.

4. Elément de retenue pour une lame de scie à oscillations selon l'une quelconque des revendications précédentes, **caractérisé en ce que** l'excentrique (36) peut être déplacé au moyen d'un levier (35), lequel est retenu dans un état verrouillé dans sa position, en particulier au moyen d'un ressort d'encliquetage (38).

5. Elément de retenue pour une lame de scie à oscillations selon l'une quelconque des revendications précédentes, l'élément de retenue étant **caractérisé en ce qu'**il comprend des moyens permettant une fixation (14) sur un arbre d'entraînement s'étendant transversalement à la direction de coupe.

6. Elément de retenue pour une lame de scie à oscillations selon l'une quelconque des revendications précédentes, **caractérisé en ce que** la partie de base (3) de la lame de scie repose par coopération de formes dans l'élément de retenue (1) dans un état verrouillé.

7. Elément de retenue pour une lame de scie à oscillations selon l'une quelconque des revendications précédentes, **caractérisé en ce qu'**une partie de base coudée (3) de la lame de scie est introduite sur toute sa longueur dans l'évidement de l'élément de retenue (1).

8. Outil à lame de scie à oscillations destiné à usiner du bois et/ou du métal, comprenant un arbre d'entraînement entraîné en oscillations et un élément de retenue (1) selon l'une quelconque des revendications précédentes.
